# EUROPEAN PATENT APPLICATION

(11) **EP 1 834 657 A1**
(43) Date of publication of application: **19.09.2007**
(21) Application number: 04821949.7
(22) Date of filing: 08.12.2004
(51) Int. Cl.: A61M 5/14

(54) **HOLDER FOR MEDICAL TREATMENT**

(71) Applicant: JMS Co., Ltd., Hiroshima-shi, Hiroshima 730-8652 (JP); JMS Singapore Pte Ltd., Singapore 569620 (SG)
(72) Inventor: YANG, Chek Lon c/o JMS SINGAPORE PTE LTD, Singapore 569620 (SG)
(74) Representative: Grape & Schwarzensteiner
(86) International application number: PCT/JP2004/018306
(87) International publication number: WO 2006/061895

(57) **Abstract**

A medical holder for assisting an operation of puncturing a flexible tube includes a first holding member 1 and a second holding member 2. The first holding member 1 and the second holding member 2 are formed so that they can be engaged with each other and can hold the flexible tube 27 therebetween when they are engaged with each other. Furthermore, a hole through which the flexible tube 27 is punctured is formed in the first holding member 1.

## Description

### Technical Field

The present invention relates to a medical holder. In particular, the present invention relates to a medical holder attached to a tube of medical equipment or a medical instrument in order to perform sampling of blood etc. or a medical fluid injection.

### Background Art

When giving a patient a blood transfusion, it is necessary to collect the patient's blood as a sample from a blood transfusion line, in order to check the compatibility with the blood to be transfused and whether or not blood coagulation occurs. When collecting blood from the blood transfusion line, it is necessary to prevent bacteria and viruses from entering the blood transfusion line. For this reason, blood collection generally is carried out via a mixture injection port or a three-way stopcock provided in the transfusion line (see Patent Document 1, for example).

Such blood collection is carried out not only when giving a patient a blood transfusion but also when giving a patient dialysis. In the case of dialysis, a mixture injection port or a three-way stopcock is provided in a blood circuit of a dialyzator.

Furthermore, when giving a patient an infusion, a medical fluid needs to be injected into an infusion line from a certain position excluding both ends of the infusion line. Also in this case, it is necessary to prevent bacteria and viruses from entering the infusion line. Thus, in the case of infusion as well, a mixture injection port or a three-way stopcock is provided in the infusion line (see Patent Document 2, for example).
Patent Document 1: JP 8(1996)-308923 A
Patent Document 2: JP 11(1999)-33124 A

### Disclosure of Invention

### Problem to be Solved by the Invention

The mixture injection port or the three-way stopcock is provided in the blood transfusion line or the infusion line by inserting a tube forming the line into the inlet and the outlet of the mixture injection port or the three-way stopcock. This, however, leads to a problem in that the position of the mixture injection port or the three-way stopcock is defined previously, thereby making it very difficult to change the position where the blood collection or the medical fluid injection is performed after starting the blood transfusion or the infusion. Accordingly, in some cases, a doctor or a medical assistant cannot perform the blood collection or the medical fluid injection from the most desirable position on the line where no other operation is hindered by the blood collection or the medical fluid injection.

Furthermore, a doctor or a medical assistant may want to perform the blood collection or the medical fluid injection from a line that is not provided with a mixture injection port or a three-way stopcock. In this case, there is no other way but to puncture the line with a needle of a syringe. However, when the line is punctured, the blood or the infusion solution may leak after pulling out the needle, and the line may be contaminated with bacteria and viruses entering from the punctured portion.

Furthermore, when the connection port of the mixture injection port or the three-way stopcock is occupied by another medical instrument, it is necessary to detach it to carry out the blood collection or the medical fluid injection. However, there is a possibility that the line might be contaminated when detaching the medical instrument.

The three-way stopcock includes a cock for converting the flow channel. When the three-way stopcock is used, it is necessary to operate the cock at the time of blood collection or medical fluid injection. However, there is a possibility that an operating error might occur.

It is an object of the present invention to solve the problems described above and to provide a medical holder that allows sampling or injection to be performed at any desired position on a line.

### Means for Solving Problem

In order to achieve the above object, the present invention provides a medical holder for assisting an operation of puncturing a flexible tube, including: a first holding member and a second holding member that are formed so that they can be engaged with each other and can hold the flexible tube therebetween when they are engaged with each other. In the first holding member, a hole through which the flexible tube is punctured is formed.

### Effects of the Invention

As specifically described above, a medical holder according to the present invention enables blood collection and medical fluid injection at a desired position on a transfusion/infusion line. Accordingly, during blood transfusion or infusion, a doctor or a medical assistant can perform blood collection or medical fluid injection from the most desirable position on the line where no other operation is hindered by the blood collection or the medical fluid injection. Moreover, the possibilities of the blood or the infusion solution leaking and of the line being contaminated with bacteria entering from the punctured portion also can be reduced. Therefore, the medical holder according to the present invention is very useful in the field of medical services.

### Brief Description of Drawings

[FIG. 1] FIG. 1 is a perspective view showing the internal structure of a medical holder according to one example of the present invention.
[FIG. 2] FIG. 2 is a perspective view showing the outer appearance of the medical holder shown in FIG. 1.
[FIG. 3] FIG. 3 is a cross-sectional view of the medical holder shown in FIG. 1, taken along line A-A' in FIG. 1.
[FIG. 4] FIG. 4 is a perspective view showing one example of an adapter as a component of a medical holder of the present invention.
[FIG. 5] FIG. 5 is a cross-sectional view showing the internal structure of the adapter shown in FIG. 4.
[FIG. 6] FIG. 6 is a cross-sectional perspective view showing a portion surrounded by the broken line in FIG. 3 in an enlarged state.
[FIG. 7] FIG. 7 is a perspective view showing the outer appearance of the medical holder with the adapter attached thereto.
[FIG. 8] FIG. 8 is a perspective view showing the state where a medical tube is held by the medical holder.
[FIG. 9] FIG. 9 is a cross-sectional view of the medical holder shown in FIG. 8.
[FIG. 10] FIG. 10 is a perspective view illustrating how a vacuum blood-collecting vessel is attached to the adapter.
[FIG. 11] FIG. 11 is a cross-sectional view showing the state where the vacuum blood-collecting vessel is attached to the adapter.
[FIG. 12] FIG. 12 is a perspective view showing another example of the adapter as a component of a medical holder of the present invention.
[FIG. 13] FIG. 13 is a perspective view showing still another example of the adapter as a component of a medical holder of the present invention.
[FIG. 14] FIG. 14 is a perspective view illustrating sampling performed using the medical holder of the present invention and a syringe.

### Description of the Invention

In the medical holder according to the present invention, it is preferable that a curved-surface portion is formed so as to fit an outer shape of the flexible tube in an engaging side region of the second holding member, whereas a pressing portion in a protrusion shape is formed in an engaging side region of the first holding member, and the flexible tube is held by being pressed by the curved-surface portion and the pressing portion. With this configuration, the medical holder can hold the flexible tube firmly

Furthermore, in the medical holder according to the present invention, it is preferable that the first holding member and the second holding member are formed integrally and are joined to each other with a bendable hinge portion. With this configuration, the medical holder can be produced easily, thereby allowing the reduction in cost.

Still further, in the medical holder according to the present invention, it is preferable that a protrusion is formed in the engaging side region of at least one of the first holding member and the second holding member, a recess or a through hole into which the protrusion fits is provided in the holding member to be engaged with the holding member having the protrusion, and the first holding member and the second holding member are engaged with each other by fitting the protrusion into the recess or the through hole. With this configuration, the first holding member and the second holding member are engaged with each other reliably.

Preferably, the medical holder according to the present invention further includes a tubular adaptor that is formed so that one end is open and the other end can be joined to the hole through which the flexible tube is punctured, and the other end of the adaptor is provided with a puncture needle for puncturing the flexible tube. With this configuration, it is possible to puncture the flexible tube easily.

Furthermore, in the foregoing configuration, it is preferable that the hole through which the flexible tube is punctured has a groove, and the other end of the adaptor has a protrusion that fits into the groove. In this case, it is possible to reduce the possibilities of the adaptor being unintentionally pulled out from the medical holder. Still further, in this configuration, it is preferable that the adaptor is formed so that a vacuum blood-collecting vessel can be inserted inside the adaptor, and a second puncture needle for puncturing the vacuum blood-collecting vessel is attached inside the adaptor so as to communicate with the puncture needle. In this case, sampling such as blood collection can be performed easily.

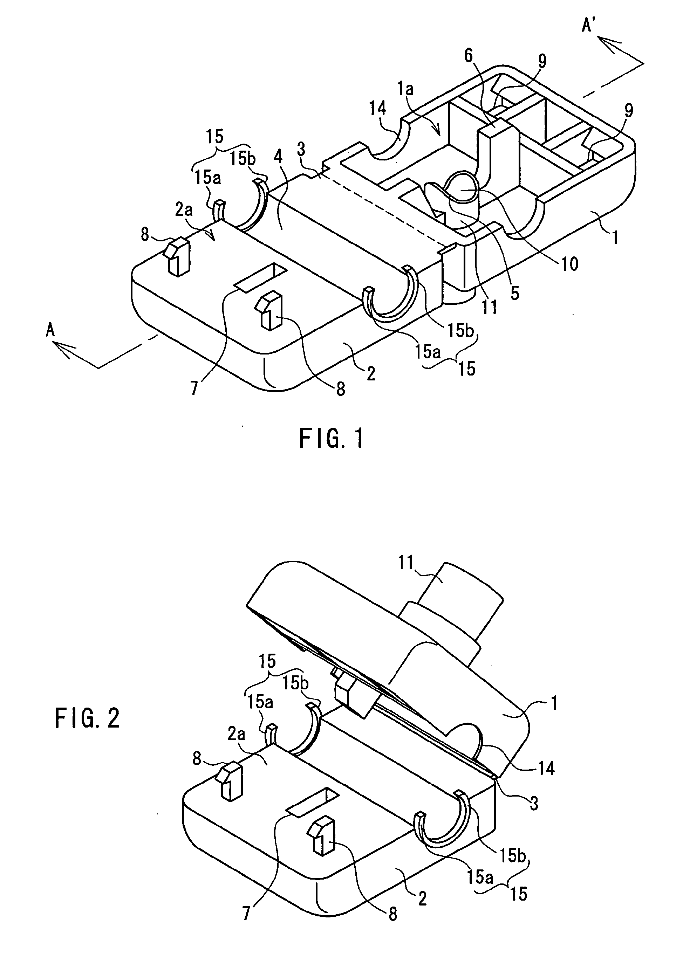

Hereinafter, a medial holder according to the present invention will be described with reference to FIGs. 1 to 11. First, the configuration of a medial holder according to one example of the present invention will be described with reference to FIGs. 1 to 6.

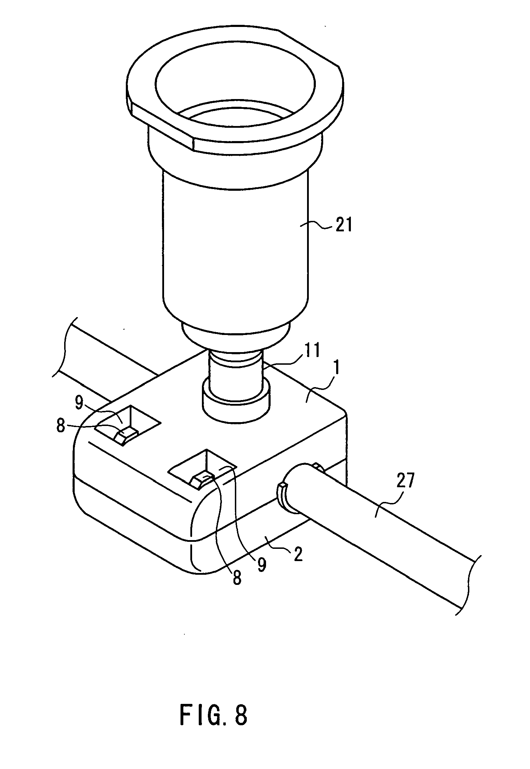

As shown in FIGs. 1 to 3, the medial holder includes a first holding member 1 and a second holding member 2. The first holding member 1 and the second holding member 2 are formed so that they can be engaged with each other. Furthermore, the first holding member 1 and the second holding member 2 hold a flexible tube (see FIGs. 8 to 10) therebetween when they are engaged with each other.

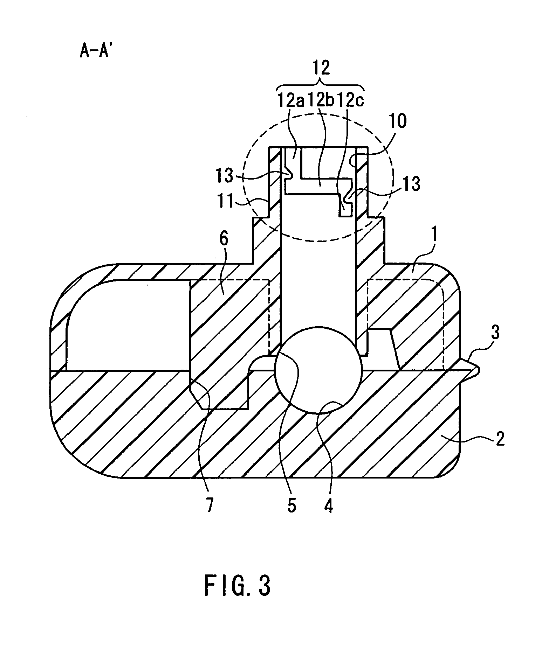

More specifically, in the present example, the first holding member 1 and the second holding member 2 are formed integrally using a resin material. Also, as can be seen from FIG. 1, the first holding member 1 and the second holding member 2 are formed so that an engaging side region 1a of the first holding member 1 and an engaging side region 2a of the second holding member 2 face toward the same direction. Furthermore, the first holding member 1 and the second holding member 2 are joined to each other with a bendable hinge portion 3. By bending the hinge portion 3, the medical holder is brought to the state as shown in FIG. 2 and then to the state as shown in FIG. 3.

Moreover, in the present example, in the engaging side region 2a of the second holding member 2, a curved-surface portion 4 is formed so as to fit the outer shape of the flexible tube. On the other hand, in the engaging side region 1a of the first holding member 1, a protrusion-shaped pressing portion 5 is formed. Accordingly, when the hinge portion 3 is bent with the flexible tube being arranged in the curved-surface portion 4, the flexible tube is pressed in the radial direction by the curved-surface portion 4 and the pressing portion 5, whereby the flexible tube is held between the curved-surface portion 4 and the pressing portion 5.

With regard to the holding of the flexible tube by the first holding member 1 and the second holding member 2, a more specific description will be provided later with reference to FIGs. 8 to 10. The shapes of the first holding member 1 and the second holding member 2 are not particularly limited as long as the flexible tube can be held between the first holding member 1 and the second holding member 2. In FIGs. 1 and 2, reference numeral 14 denotes a notch. The notches 14 are formed so as to fit the outer shape of the flexible tube in order to prevent the flexible tube from being pressed excessively.

The curved-surface portion 4 is formed so as to extend from one side face to the other side face of the second holding member 2. At each end of the curved-surface portion 4, a retaining portion 15 for retaining the flexible tube arranged in the curved-surface portion 4 is formed. The retaining portion 15 includes a pair of protruding portions 15a and 15b. The protruding portions 15a and 15b are formed so that their front ends are close to each other in order to reduce the possibilities that the flexible tube might be unintentionally pulled out from the medical holder.

Furthermore, in the present example, a protrusion 6 is provided in the engaging side region 1a of the first holding member 1. On the other hand, in the engaging side region 2a of the second holding member 2 that is to be engaged with the first holding member 1, a recess 7 into which the protrusion 6 can fit is provided. Furthermore, two protrusions 8 are provided in the engaging side region 2 a of the second holding member 2, and two through holes 9 into which the respective protrusions 8 can fit are provided in the first holding member 1 that is to be engaged with the second holding member 2.

Thus, when the hinge portion 3 is bent, the protrusion 6 is fitted into the recess 7, and the protrusions 8 are fitted into the through holes 9, respectively. As a result, the state as shown in FIG. 3 is maintained. In the present example, the first holding member 1 and the second holding member 2 are engaged with each other by fitting the protrusion 6 into the recess 7 and the protrusions 8 into the through holes 9, respectively. The protrusions 8 are formed in a hook shape so as to prevent them from being unintentionally pulled out from the through holes 9.

Furthermore, in the first holding member 1, a hole 10 through which the flexible tube being held is punctured is formed, as shown in FIGs. 1 and 3. In the present example, as shown in FIGs. 1 to 3, a tubular member 11 extending in the thickness direction of the first holding member 1 is formed, and the inner space of this tubular member 11 serves as the hole 10. The front end of the tubular member 11 serves as the pressing portion 5 for pressing the flexible tube. Furthermore, on the inner wall of the tubular member 11, a groove 12 is formed, which is used when attaching an adaptor to be described later.

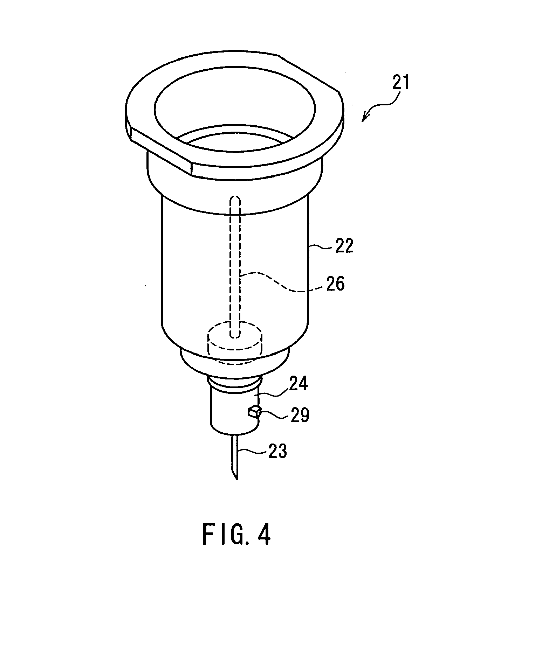

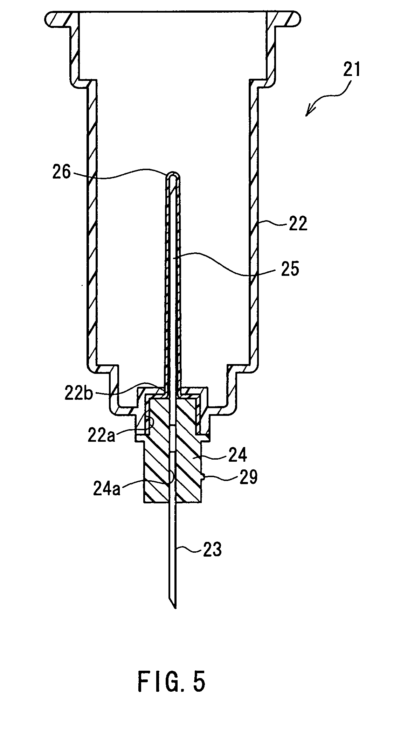

The medical holder according to the present example includes, in addition to the first holding member 1 and the second holding member 2, an adaptor 21 shown in FIGs. 4 and 5. The adaptor 21 shown in FIGs. 4 and 5 is used for puncturing the flexible tube, and is connected to a vacuum blood-collecting vessel (not shown), for example.

As shown in FIGs. 4 and 5, the adaptor 21 is formed in a tubular shape with its one end open. The other end of the adaptor 21 is provided with a puncture needle 23 for puncturing the flexible tube. Furthermore, the other end of the adaptor 21 is formed so that it can be inserted into the hole 10 (see FIGs. 1 and 3) and joined thereto.

More specifically, in the present example, the adaptor 21 includes a main body 22 having a tubular shape with its one end open. The main body 22 is formed so that a vacuum blood-collecting vessel can be inserted therein. On the other end of the main body 22, a recess 22a is provided. A holding member 24 for attaching the puncture needle 23 to the main body 22 is fitted into the recess 22a. At the bottom of the recess 22a, a through hole 22b is provided.

Furthermore, in the present example, a through hole 24a penetrating the holding member 24 is formed in the holding member 24 so as to communicate with the through hole 22b formed in the recess 22a. The puncture needle 23 is inserted into the through hole 24a of the holding member 24. Into the through hole 24a, a second puncture needle 25 also is inserted. The second puncture needle 25 communicates with the puncture needle 23 and protrudes inside the main body 22 from the thought hole 22b.

In FIGs. 4 and 5, reference numeral 26 denotes a cover (made of an elastomer, for example) for protecting the second puncture needle 25. Furthermore, the adaptor 21 is connected to a vacuum blood-collecting vessel.

Furthermore, in the present example, the portion of the holding member 24 protruding from the main body 22 has an outer shape that can be joined to the hole 10 (see FIGs. 1 and 3). Moreover, a protrusion 29 is formed on this protruding portion. The protrusion 29 fits into the groove 12 formed on the inner wall of the tubular member 11 of the first holding member 1.

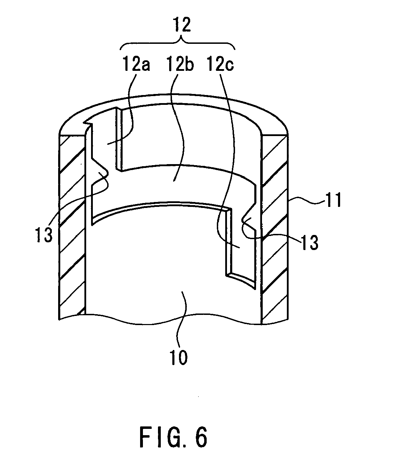

Hereinafter, how the adaptor 21 (see FIGs. 4 and 5) is attached to the hole 10 (see FIGs. 1 and 3) will be described with reference to FIG. 6. On the inner wall of the tubular member 11, which forms the hole 10, the groove 12 is formed as shown in FIGs. 3 and 6. The groove 12 includes a first groove 12a and a third groove 12c extending in the puncture direction and a second groove 12b extending in the circumferential direction of the hole 10.

Furthermore, the first groove 12a, the second groove 12b, and the third groove 12c are arranged from the outer opening (i.e., the opening on the adapter side) toward the inner opening of the tubular member 11 in this order. The first groove 12a is formed so that it links the outer opening (i.e., the opening on the adapter side) of the tubular member 11 to one end of the second groove 12b. The third groove 12c is formed so as to be linked to the other end of the second groove 12b.

In the present example, protrusions 13 protruding toward the inner portion of the groove 12 are formed. The protrusions 13 are provided in order to reduce the possibilities of the protrusion 29 provided on the holding member 24 being unintentionally detached from the groove 12.
Furthermore, the lengths of the first groove 12a and the third groove 12c are adjusted so that the front end of the puncture needle 23 attached to the adaptor 21 would be placed in an inner space of the flexible tube.

The adaptor 21 (see FIGs. 4 and 5) can be joined to the hole 10 (see FIGs. 1 and 3) in the following manner. First, the protrusion 29 (see FIGs. 4 and 5) provided on the holding member 24 is lined up with the first groove 12a (see FIGs. 3 and 6). In this state, the adaptor 21 is pushed in the puncture direction. Subsequently, the adaptor 21 is turned clockwise until it stops. Thereafter, the adaptor 21 is pushed further in the puncture direction.

As a result, the adaptor 21 is joined firmly to the hole 10 so that it cannot be detached easily. At the same time, the positioning of the puncture needle 23 attached to the adaptor 21 also is accomplished.

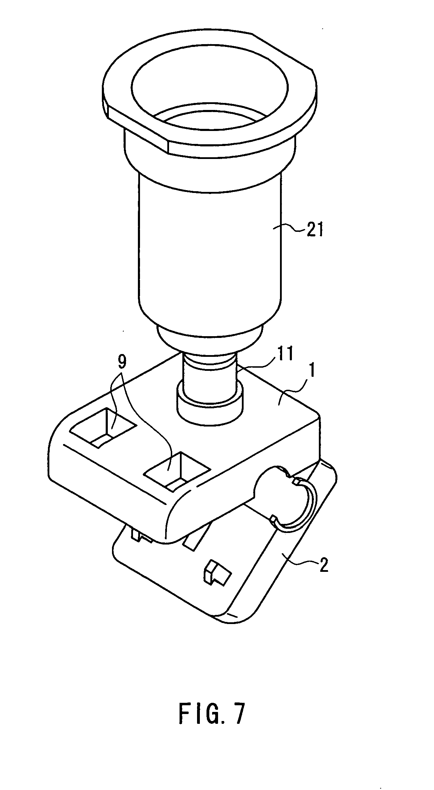

Next, blood sampling using the medical holder shown in FIGs. 1 to 6 will be described with reference to FIGs. 7 to 11. In the following description, FIGs. 1 to 6 will be referred to as necessary.

First, as shown in FIG. 7, the adaptor 21 (see FIGs. 4 and 5) is joined to the hole 10 of the medical holder (see FIGs. 1 to 3). The adaptor 21 is joined to the hole 10 by fitting the protrusion 29 of the adaptor 21 into the groove 12 of the hole 10, as described above with reference to FIG. 6.

Next, as shown in FIG. 8, a desired portion of a flexible tube 27 is held between the first holding member 1 and the second holding member 2. In the present example, the flexible tube 27 is a medical tube forming a blood transfusion line and is connected to a blood bag or the like. Moreover, the portion of the flexible tube 27 to be held between the first holding member 1 and the second holding member 2 may be the most convenient position for a doctor or a medical assistant. Preferably, the portion of the flexible tube 27 to be held between the first holding member 1 and the second holding member 2 is sterilized, for example, by being wiped with cotton moistened with alcohol.

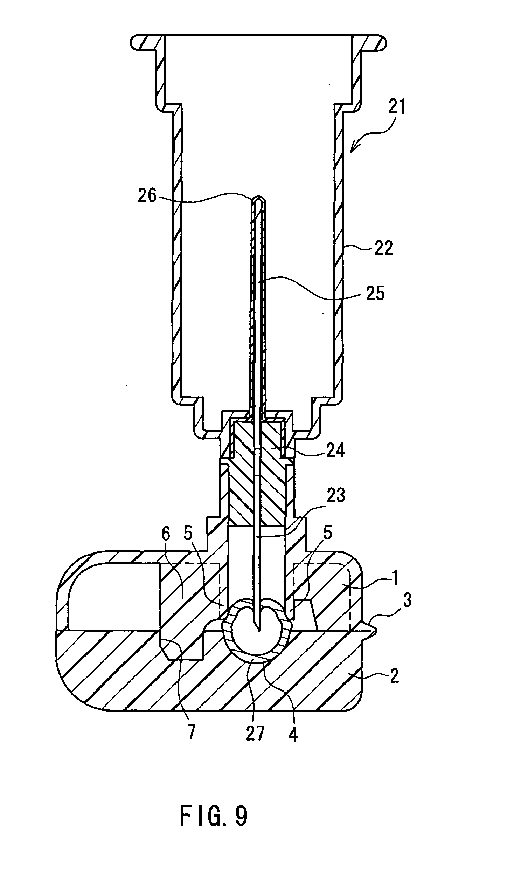

More specifically, as described above with reference to FIGs. 1 to 3, the hinge portion 3 is bent with the flexible tube 27 arranged in the curved-surface portion 4, thereby engaging the first holding member 1 and the second holding member 2 with each other. As a result, as shown in FIG. 9, the flexible tube 27 is pressed in the radial direction by the curved-surface portion 4 and the pressing portion 5 so that it cannot be detached easily. At this time, the puncture needle 23 pierces through the side wall of the flexible tube 27 to go into the inner space of the flexible tube 27.

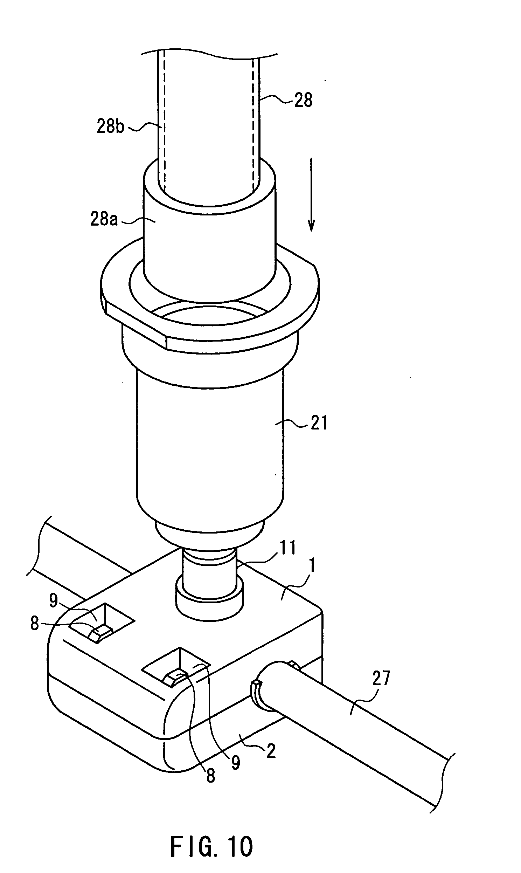

Next, as shown in FIG. 10, a vacuum blood-collecting vessel 28 is inserted inside the tubular main body 22 of the adaptor 21. The vacuum blood-collecting vessel 28 includes a glass tube 28b with its one end being closed and a rubber cap 28a for sealing the opening at the other end of the glass tube 28b. The vacuum blood-collecting vessel 28 is inserted into the tubular main body 22 of the adaptor 21 with the cap 28a facing the second puncture needle 25.

When the vacuum blood-collecting vessel 28 is inserted fully, the second puncture needle 25 pierces through the cover 26 and the cap 28a of the vacuum blood-collecting vessel 28 to go into the inner space of the vacuum blood-collecting vessel 28. At this time, the pressure inside the vacuum blood-collecting vessel 28 is lower than that inside the flexible tube 27. Accordingly, a part of the blood flowing through the flexible tube 27 is drawn to the vacuum blood-collecting vessel 28 via the puncture needle 23 and the second puncture needle 25.

As specifically described above, the medical holder according to the present invention can be provided at any desired position on the blood transfusion line later without providing a mixture injection port or a three-way stopcock on the blood transfusion beforehand. Therefore, by using the medical holder of the present invention, blood sampling can be performed at any desired position on the blood transfusion line that already has been constructed. This allows a doctor or a medical assistant to perform blood collection from the most desirable position on the line where no other operation is hindered by the blood collection.

Moreover, since the puncture needle 23 is fixed in the flexible tube 27 by the adaptor 21 and the medical holder, it is not necessary to pull out the puncture needle 23 from the flexible tube 27 before the transfusion is completed.

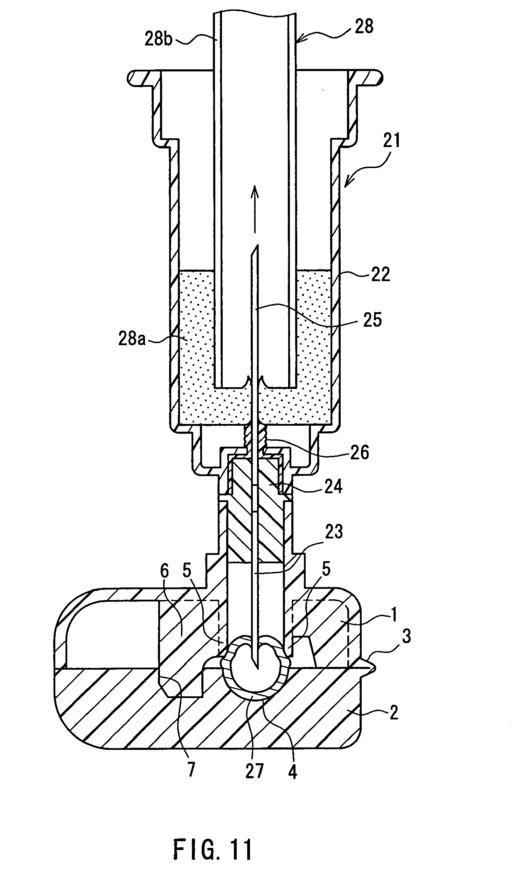

Furthermore, after the vacuum blood-collecting vessel 28 has been pulled out, the cover 26 returns to its original form and covers the second puncture needle 25 again. At this time, the front end of the cover 26 has been broken by the second puncture needle 25 (see FIG. 11). However, in this example, since the cover 26 is made of an elastomer, the broken portion is closed when the cover 26 returns to its original form. Thus, it is possible to ensure a sealed state between the second puncture needle 25 and the cover 26.

For the above-described reasons, the possibilities that the blood might leak or the flexible tube 27 might be contaminated due to the puncture of the flexible tube 27 with the puncture needle 23 can be reduced.

When a pressure is applied within the flexible tube 27 in the direction to expand the flexible tube 27 (i.e., a positive pressure), there is a risk that the blood might leak from the portion punctured with the puncture needle 23. In this case, the blood that has leaked from the punctured portion may leak to the outside of the medical holder from a space between the holding member 24 of the adaptor 21 and the hole 10, between each of the notches 14 of the first holding member 1 and the flexible tube 27, between the curved-surface portion 4 and the flexible tube 27, between each of the protrusions 8 of the first holding member 1 and each of the through holes 9 of the second holding member 2, or the like. Furthermore, when the blood that has leaked from the punctured portion is infected with viruses or bacteria, the ambient environment and medical workers may be contaminated with the blood when it leaks to the outside of the medical holder.

In order to prevent the occurrence of such situation, a liquid absorbing member preferably is disposed in an inner space formed by engaging the first holding member 1 and the second holding member 2 with each other. Furthermore, the holding member 24 preferably is formed in such a manner that an outer surface of the portion of the holding member 24 protruding from the main body 22 is in intimate contact with the inner surface of the hole 10. Still further, it is preferable that the notches 14 of the first holding member 1 and the curved-surface portion 4 of the second holding member 2 are formed in such a manner that the notches 14 and the curved-surface portion 4 as a whole are in intimate contact with the flexible tube 27.

While FIGs. 1 to 11 are directed to an example where the first holding member 1 and the second holding member 2 are formed integrally, the present invention is not limited thereto. In the present invention, the first holding member 1 and the second holding member 2 may have any configurations as long as they can be engaged with each other, and thus they may be separate components. Furthermore, the shape, size, or material of the first holding member 1 and a second holding member 2 is not particularly limited.

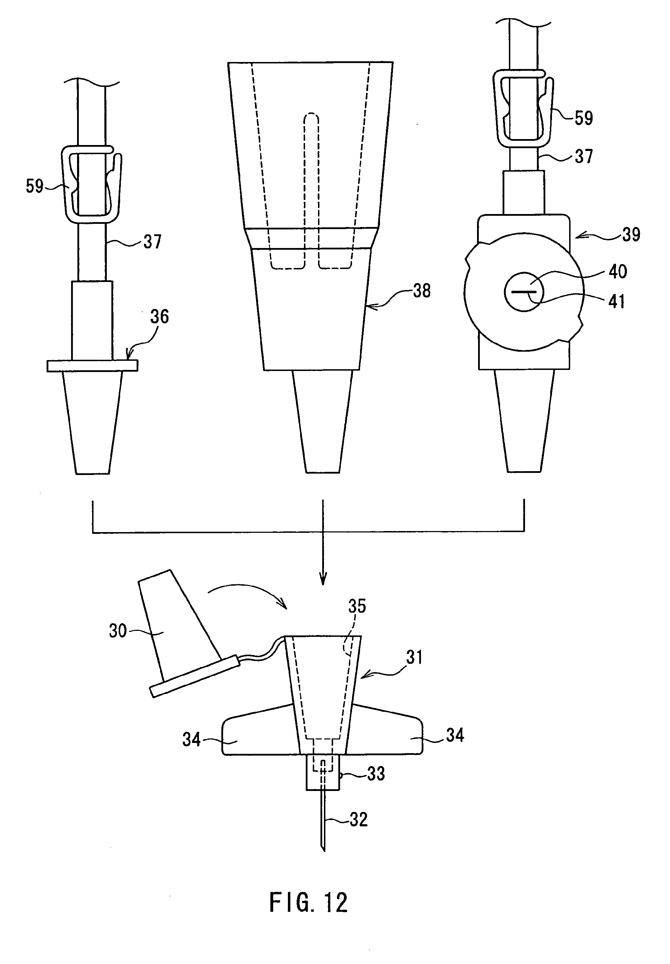

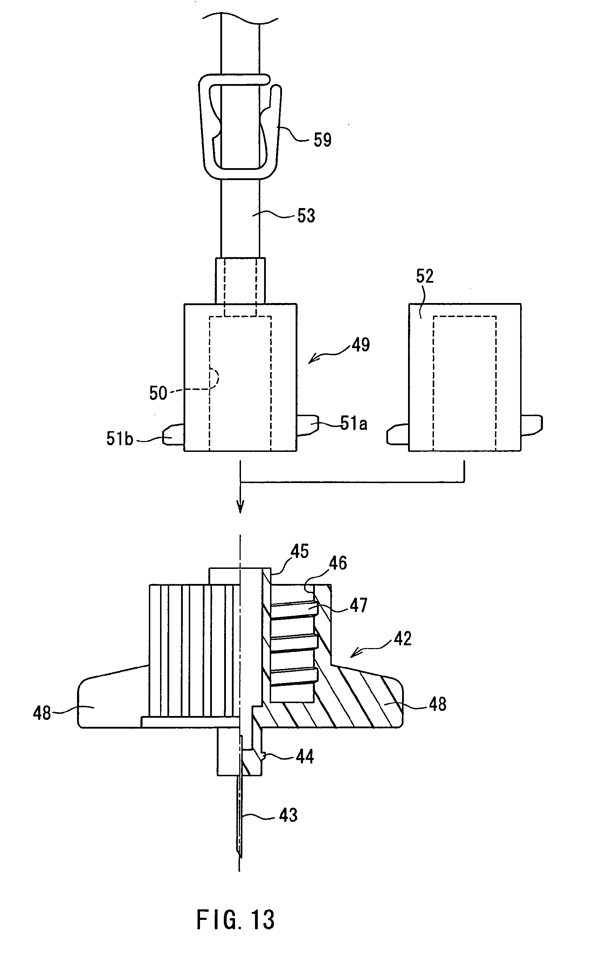

In the present invention, an adaptor to be attached to the first holding member 1 and the second holding member 2 is not limited to that shown in FIGs. 4 and 5. FIGs. 12 and 13 show further examples of the adaptor. Also, the adaptor shown in FIG. 11 of WO 00/63088 is a still further example of the adaptor to be attached to the first holding member 1 and the second holding member 2.

Similarly to the adaptor 21 shown in FIGs. 4 and 5, an adaptor 31 shown in FIG. 12 also is provided with a puncture needle 32 for puncturing the flexible tube being held (see FIGs. 8 to 11). On the front end of the adaptor 31, a protrusion 33 to be joined to the groove 12 (see FIG. 6) formed on the inner wall of the hole 10 of the first holding member 1 is provided. Reference numeral 34 denotes a wing-like part provided for allowing the adaptor 31 to be joined to the hole 10 easily.

Note here that the adaptor 31 shown in FIG. 12 differs from the adaptor 21 shown in FIGs. 4 and 5 in that it is not designed only for the connection to a vacuum blood-collecting vessel. The adaptor 31 is provided with a tapered insertion hole 35. The insertion hole 35 is a female Luer connector, and the adaptor 31 is used for the connection to a medical instrument provided with a male Luer connector.

When the medical instrument provided with a male Luer connector is not connected to the adaptor 31, or when detaching the medical instrument connected thereto, it is preferable to close the insertion hole 35 so that the entry of various substances from the outside can be inhibited. On this account, the adaptor 31 preferably is provided with a cap 30 that can fit into the insertion hole 35, as shown in FIG. 12.

Examples of the medical instrument provided with a male Luer connector include those shown in the upper part of FIG. 12. Reference numeral 36 denotes a joint to which a tube 37 is attached. A male Luer connector that can be joined to the insertion hole 35 is formed at the front end of the joint 36.

Reference numeral 38 denotes an adaptor for attaching a vacuum blood-collecting vessel. Inside the adaptor 38, a needle (not shown) for puncturing a vacuum blood-collecting vessel is provided. Furthermore, a male Luer connector that can be joined to the insertion hole 35 also is formed at the front end of the adaptor 38. By inserting the front end of the adaptor 38 into the insertion hole 35 of the adaptor 31, the blood collection can be performed in the same manner as in the case of the adaptor 21 shown in FIGS. 4 and 5.

Reference numeral 39 denotes a mixture injection port. The mixture injection port 39 is provided with a valve 40. The valve 40 is formed of a round septum that has a slit 41 in its center so that a medical instrument is inserted thereinto. Since the septum forming the valve 40 is made of an elastic material, the sealing state inside the mixture injection port 39 can be maintained even if a medical instrument inserted in the slit 41 is pulled out. The tube 37 also is attached to the mixture injection port 39.

Furthermore, as shown in FIG. 12, the tube 37 attached to the joint 36 and the mixture injection port 39 is provided with a clamp 59. The clamp 59 is provided for closing the flow channel when the joint 36 or the mixture injection port 39 is not used.

An adaptor 42 shown in FIG. 13 also is provided with a puncture needle 43 for puncturing the flexible tube being held, similarly to the adaptor 21 shown in FIGs. 4 and 5. On the front end of the adaptor 42, a protrusion 44 to be joined to the groove 12 (see FIG. 6) formed on the inner wall of the hole 10 of the first holding member 1 is provided.

Note here that the adaptor 42 is designed for threaded engagement with a tubular joint 49. The adaptor 42 has a recess 46 to be joined to an outer surface of the joint 49 and a tubular member 45 to be inserted into a hole 50 inside the joint 49. Furthermore, a helical groove 47 is formed on the side wall of the recess 46. In FIG. 13, only the right-half of the adaptor 42 is shown as a cross-sectional view.

On the outer surface of the joint 49, a pair of protrusions 51a and 51b that fit in the groove 47 is formed. Thus, by aligning the front end of the tubular member 45 with the opening of the hole 50 and then screwing the joint 49, the protrusions 51a and 51b fit in the groove 47, thereby achieving threaded engagement between the joint 49 and the adaptor 42. The outer surface of the joint 49 may have a helical protrusion that fits into the groove 47 instead of the pair of protrusions 51a and 51b.

A tube 53 is connected to the joint 49. As in the case of the example shown in FIG. 12, the tube 53 also is provided with a clamp 59. Furthermore, when the joint 49 is not connected to the adaptor 42, or when detaching the joint 49 connected thereto, a cap 52 is attached to the adaptor 42, as in the case of the adaptor 31 shown in FIG. 12. The cap 52 also is attached to the adaptor 42 via the threaded engagement, similarly to the joint 49.

In the example shown in FIG. 13, the joint 49 may be in any form as long as it is connected to the adaptor 42 via threaded engagement or locking engagement by means of the pair of protrusions 51a and 51b or a helical protrusion, although such further examples are not shown in the drawing. For example, the joint 49 may be provided with a male Luer connector, similarly to the adaptor 31 shown in FIG. 12. Alternatively, the joint 49 may be configured so that a vacuum blood-collecting vessel can be attached thereto, similarly to the adaptor 38 shown in FIG. 12. Furthermore, the joint 49 may be provided with a valve into which a medial instrument can be inserted, similarly to the mixture injection port 39 shown in FIG. 12.

While FIGs. 7 to 11 are directed to an example where blood sampling is performed using a medical holder according to the present invention and the vacuum blood-collecting vessel 28, the present invention is not limited thereto. For example, a medical holder according to the present invention may be used for injecting a medical fluid into an infusion line. This can be achieved, for example, by using the adaptor 31 and the mixture injection port 39 shown in FIG. 12. More specifically, any desired position on the infusion line may be held by the medical holder of the present invention, and then a medical fluid may be injected into the infusion line via the mixture injection port 39.

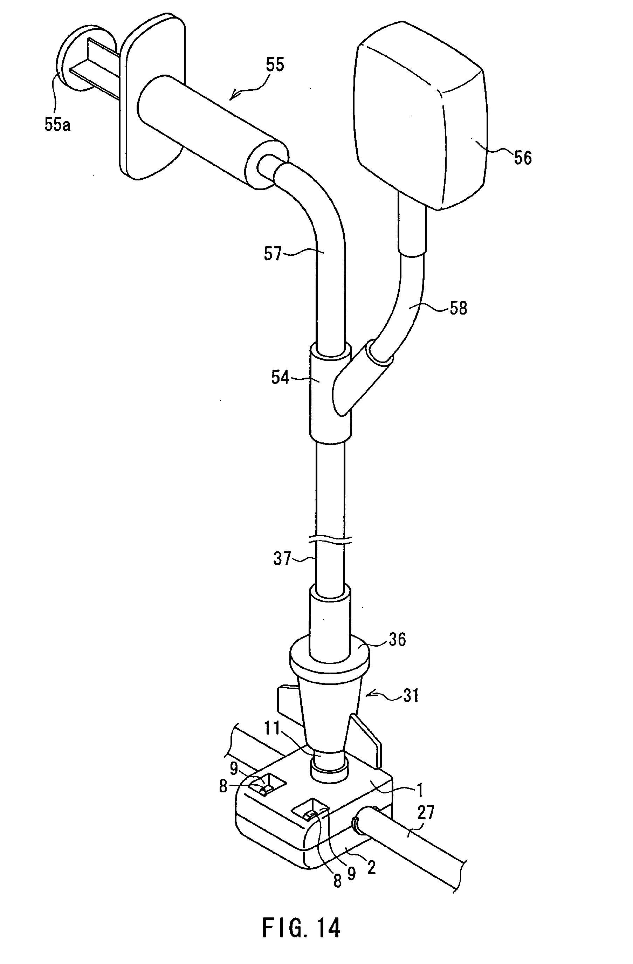

Moreover, the medical holder according to the present invention also can be used for sampling that is performed using a syringe. This will be described with reference to FIG. 14. Unlike the examples shown in FIGs. 7 to 11, the adaptor 31 shown in FIG. 12 is used in the example shown in FIG. 14. To the adaptor 31, the joint 36 shown in FIG. 12 is connected.

As shown in FIG. 14, one of the opposing connection ports of a Y-shaped pipe 54 is connected to the tube 37 that is connected to the joint 36, and the other of the opposing connection ports is connected to a syringe 55 via a tube 57. Furthermore, the connection port of the branch portion of the Y-shaped pipe 54 is connected to a sample bag 56 via a tube 58.

With the foregoing configuration, the liquid flowing through the flexible tube 27 can be drawn by pulling a piston 55a of the syringe 55 to generate a negative pressure in the syringe 55. The liquid thus drawn is collected in the sample bag 56. The liquid that has flown into the syringe 55 can be sent to the sample bag 56 by pressing the piston 55a in the state where the tube 37 is clamped.

### Industrial Applicability

A connector and a connecting structure according to the present invention can be used without any limitation in technical fields in which connection of tubular members is required. In particular, the connector and the connecting structure according to the present invention are useful in the filed of medicine, in which there is a strong demand for highly reliable connection and prevention of liquid leakage.

## Claims

1. A medical holder for assisting an operation of puncturing a flexible tube, comprising:
a first holding member and a second holding member that are formed so that the first holding member and the second holding member can be engaged with each other and can hold the flexible tube between the first holding member and the second holding member when the first holding member and the second holding member are engaged with each other,
wherein a hole through which the flexible tube is punctured is formed in the first holding member.

2. The medical holder according to claim 1, wherein
a curved-surface portion is formed so as to fit an outer shape of the flexible tube in an engaging side region of the second holding member, whereas a pressing portion in a protrusion shape is formed in an engaging side region of the first holding member, and
the flexible tube is held by being pressed by the curved-surface portion and the pressing portion.

3. The medical holder according to claim 1 or 2, wherein the first holding member and the second holding member are formed integrally and are joined to each other with a bendable hinge portion.

4. The medical holder according to any one of claims 1 to 3, wherein
a protrusion is formed in the engaging side region of at least one of the first holding member and the second holding member,
a recess or a through hole into which the protrusion fits is provided in the holding member to be engaged with the holding member having the protrusion, and
the first holding member and the second holding member are engaged with each other by fitting the protrusion into the recess or the through hole.

5. The medical holder according to any one of claims 1 to 4, further comprising a tubular adaptor that is formed so that one end is open and the other end can be joined to the hole through which the flexible tube is punctured,
wherein the other end of the adaptor is provided with a puncture needle for puncturing the flexible tube.

6. The medical holder according to claim 5, wherein the hole through which the flexible tube is punctured has a groove, and the other end of the adaptor has a protrusion that fits into the groove.

7. The medical holder according to claim 5 or 6, wherein the adaptor is formed so that a vacuum blood-collecting vessel can be inserted inside the adaptor, and a second puncture needle for puncturing the vacuum blood-collecting vessel is attached inside the adaptor so as to communicate with the puncture needle.
